Europäisches Patentamt

**European Patent Office**

Office européen des brevets

(11) Veröffentlichungsnummer: **0 209 155 B1**

(12) **EUROPÄISCHE PATENTSCHRIFT**

(45) Veröffentlichungstag der Patentschrift:
07.11.90

(21) Anmeldenummer: 86109900.0

(22) Anmeldetag: 18.07.86

(51) Int. Cl.⁵: **G01N 33/535**, C12Q 1/28, **G01N 33/78**

(54) Konjugat für die Enzymimmunobestimmung.

(30) Priorität: 19.07.85 DE 3525911

(43) Veröffentlichungstag der Anmeldung:
21.01.87 Patentblatt 87/4

(45) Bekanntmachung des Hinweises auf die Patenterteilung:
07.11.90 Patentblatt 90/45

(84) Benannte Vertragsstaaten:
AT BE CH DE FR GB IT LI LU NL SE

(56) Entgegenhaltungen:
EP-A- 0 084 807
DE-A- 3 136 579
US-A- 4 486 534

K. Lübke und B. Nieuweboer "Immunologische Teste für niedermolekulare Wirkstoffe", 1978 GEORG THIEME VERLAG, Stuttgart Seite 118

(73) Patentinhaber: BOEHRINGER MANNHEIM GMBH,
Sandhofer Strasse 116, D-6800 Mannheim 31(DE)

(72) Erfinder: Haug, Harald, Dr. rer. nat., Kreuzeckstrasse 12,
D-8123 Peissenberg(DE)
Erfinder: Kleinhammer, Gerd, Dr. rer. nat.,
Kreuzeckstrasse 3, D-8132 Tutzing(DE)
Erfinder: Mattersberger, Johann, Dr. phil.,
Oefelestrasse 5, D-8000 München 90(DE)

(74) Vertreter: Weickmann, Heinrich, Dipl.-Ing. et al,
Patentanwälte Dipl.-Ing. H.Weickmann Dipl.-Phys.Dr.
K.Fincke Dipl.-Ing. F.A.Weickmann Dipl.-Chem. B. Huber
Dr.-Ing. H. Liska Dipl.-Phys.Dr. J. Prechtel
Postfach 860820, D-8000 München 86(DE)

## Beschreibung

Immunologische Nachweismethoden haben für viele analytische Zwecke, insbesondere aber im Rahmen der klinischen Analyse, aufgrund ihrer außerordentlichen Empfindlichkeit und Spezifität überragende Bedeutung erlangt und die rein chemischen Methoden in den Hintergrund gedrängt. Bei diesen Analysenverfahren wird in der Regel einer der immunologischen Bindungspartner markiert, wobei vor allem die radioaktive Markierung (RIA) zu Beginn und später die Enzymmarkierung (EIA) hervorragende Bedeutung erlangt haben. Im Rahmen des immunologischen Nachweisverfahrens unter Verwendung der Enzymmarkierung hat sich wiederum eine kleine Anzahl von Enzymen als besonders geeignet für Markierungszwecke erwiesen, wobei leichte Nachweisbarkeit, hohe Stabilität und möglichst geringe Beeinflussung der Immunreaktion die für die Bevorzugung maßgebenden Eigenschaften sind. Zu den bereits verwendeten oder verwendbaren Markierungsenzymen gehören Glucoseoxidase (GOD) E.C. 1.1.3.4, β-Fructosidase (Invertase) E.C. 3.2.1.26, alkalische Phosphatase (AP) E.C. 3.1.3.1 und Peroxidase (POD) E.C. 1.11.1.7, die alle einen Kohlenhydratanteil im Molekül enthalten. Sie werden in Form von sogenannten Enzymkonjugaten eingesetzt. Hierunter werden Kupplungsprodukte der Markierungsenzyme mit einem der immunologisch wirksamen Reaktionspartner verstanden, wobei in der Regel eine kovalente Bindung zwischen dem Markierungsenzym und der immunologischen Kupplungskomponente, die beispielsweise ein Antigen, ein Hapten, ein Antikörper, ein Derivat oder Fragment eines Antikörpers sein·kann, besteht.

Aus der DE-A 3 136 579 ist es bekannt, ein Thyroxin-Peroxidase (POD)-Konjugat nach der Methode von Nakane und Kawaoi, J. Histochem. Cytochem. 22, 1984, (1974) herzustellen, wobei die kohlehydrathaltige Komponente des Glykoproteins POD mit einem Hapten ($T_4$) so gekuppelt wird, daß in einem ersten Schritt zuerst die Aminogruppe des POD mit Fluorescein-Isothiocyanat blockiert und dann mit Perjodat oxidiert wird. Auf diese Weise werden reaktive Aldehydgruppen erhalten, die anschließend mit den freien Aminogruppen des $T_4$ gekoppelt werden. Die durch diese Kopplungsreaktion entstandenen Bindungen sind Schiff'sche Basen, die anschließend wieder reduktiv stabilisiert werden müssen. Man erhält so ein POD-Konjugat, welches mehrere $T_4$-Moleküle enthält, die jeweils über die Zuckerreste an das Enzym gebunden sind. Eine Oxidations-Reduktionsbehandlung ist bei diesem Verfahren daher eine unabdingbare Voraussetzung, um eine stabile Bindung zwischen den Konjugationspartnern zu erzielen.

Auch die US-A 4 486 534 offenbart eine Kupplungsreaktion nach dem Nakane-Verfahren. Dabei wird in einem ersten Schritt das Glykoprotein TBG mit β-Gal als zweitem Protein dadurch gekoppelt, daß in einem ersten Schritt im TBG mittels einer Perjodatperoxidation reaktive Aldehyde erzeugt werden, die anschließend mit den Aminogruppen der β-Gal gekoppelt und durch Reduktion mit Borhydrid stabilisiert werden. Man erhält so ein Vernetzungsprodukt von TBG mit β-Gal. Auch bei diesem Verfahren ist daher ein Oxidations- und ein Reduktionsschritt Teil der Kupplungsreaktion selbst.

Es hat sich nun gezeigt, daß zwar bei der weit überwiegenden Mehrzahl der Seren, bei denen ein interessierender Bestandteil durch eine EIA-Methode unter Verwendung von derartigen Markierungsenzymen, insbesondere von POD als Markierungsenzym bestimmt wird, richtige Ergebnisse erhalten werden, wenn man mit den in einem entsprechenden RIA-System erhaltenen Resultaten vergleicht. Bei einer gewissen Anzahl von Seren, die im folgenden als Problemseren bezeichnet werden, ergab sich aber eine deutliche Abweichung von den richtigen Werten in Form von zu hoch positiven Resultaten.

Der Erfindung liegt daher die Aufgabe zugrunde, diesen Nachteil zu beseitigen und Konjugate von Markierungsenzymen für den Enzymimmunoassay (EIA) zur Verfügung zu stellen, welche auch bei den sogenannten Problemseren richtige Resultate liefern.

Gelöst wird diese Aufgabe erfindungsgemäß durch ein Verfahren zur Herstellung eines für die Enzymimmunobestimmung geeigneten Konjugats aus einem Markierungsenzym, welchen einen Kohlenhydratanteil enthält, wie Peroxidase, und einer immunologisch wirksamen Substanz unter Anwendung einer am Proteinanteil des Markierungsenzyms angreifenden Kupplungsmethode, welches dadurch gekennzeichnet ist, daß man das Markierungsenzym vor oder nach der Kupplung mit der immunologisch wirksamen Substanz mit Perjodsäure oder einem Alkalisalz derselben in wäßrigem Medium oxidiert und das Oxidationsprodukt dann mit $NaBH_4$ reduziert.

Überraschenderweise hat sich herausgestellt, daß eine Perjodatoxidation des Markierungsenzym-Moleküls mit anschließender Reduktion mit $NaBH_4$ ein Markierungsenzym-Derivat liefert, welches seine enzymatischen Eigenschaften unverändert beibehält, aber auch mit den sogenannten Problemseren richtige Werte liefert, die mit den nach RIA-Methoden erhaltenen Werten übereinstimmen.

Das Verfahren der Erfindung wird hinsichtlich der pH-Werte, Auswahl der geeigneten Puffer und Pufferkonzentration unter den dem Fachmann bekannten Bedingungen für die Aufrechterhaltung der Enzymaktivität durchgeführt. Bevorzugt erfolgt im Falle von POD der Oxidationsschritt bei pH-Werten zwischen 4 und 8,5 in gepufferter Lösung. Die Reduktion bewirkt man vorzugsweise bei pH-Werten zwischen 7,5 und 9. In jedem Fall ist es zweckmäßig, die Umsetzungen unter Kühlung durchzuführen, insbesondere bei Temperaturen unter 10°C, vorzugsweise zwischen 0 und 5°C. Prinzipiell ist es aber auch möglich, angesichts der bekanntlich relativ guten Temperaturstabilität der POD auch bei höheren Temperaturen bis zu etwa 37° zu arbeiten. Für die anderen, im Rahmen der Erfindung geeigneten Enzyme gelten die ihren bekannten Eigenschaften entsprechenden Bedingungen hinsichtlich pH-Wert und Temperatur.

Da die oben erwähnten falschen Resultate vor allem bei Konjugaten auftraten, bei denen Enzym mit einem Hapten verknüpft vorliegt, wird angenommen, daß die Gefahr falscher Resultate besonders dann besteht, wenn extreme Größenrelationen zwischen dem Markierungsenzym und seinem Bindungspartner vorliegen, letzterer also, verglichen mit dem Enzym-Molekül, sehr klein ist. Ein typisches Beispiel hierfür sind Thyroxin (T4) und Trijodthyronin (T3), also niedermolekulare hormonal wirksame Haptene als Bindungspartner. Überraschenderweise gelingt es erfindungsgemäß, falsche, zu hohe Resultate zu verhindern, obwohl dabei das Größenverhältnis der beiden Bindungspartner im Konjugat unverändert bleibt, d. h. das große Enzym-Molekül unverändert gegenüber seinem kleinen Bindungspartner eine sterische Abschirmwirkung entfalten kann.

Die Kupplung der Enzyme kann im Rahmen des erfindungsgemäßen Verfahrens vor der erfindungsgemäßen Oxidations-/Reduktionsbehandlung oder auch danach durchgeführt werden. Eine Durchführung der erfindungsgemäßen Oxidations-/Reduktionsbehandlung nach Herstellung des Konjugats ist vor allem auch dann möglich, wenn der immunologisch wirksame Bindungspartner selbst ein Protein ist, beispielsweise TBG oder ein Antikörper oder Antikörperfragment.

Für die Herstellung des erfindungsgemäßen Markierungsenzym-Konjugats eignen sich die üblichen Kupplungsmethoden, soweit sie nicht selbst eine Oxidation beinhalten. Geeignete Methoden sind beispielsweise beschrieben in J. of Immunoassay, Vol. 4(3), 209-327 (1983). Als besonders geeignet erwiesen sich Kupplungsmethoden unter Verwendung von bifunktionellen Reagenzien, welche die Verknüpfung von Aminogruppen oder Sulfhydrylgruppen bewirken. Bevorzugte Beispiele für derartige bifunktionelle Reagenzien sind N-Succinimidyl-4-(N-maleimidomethyl)- cyclohexan-1-carboxylat (hinge-Methode) und Glutardialdehyd (Immunochem. 8, 1175-1179 (1971)). Auch die direkte Kondensation unter Verwendung von Carbodiimid (J. Cell. Biol. 33, 307-318 (1967)) kann angewendet werden. Generell sind als bifunktionelle Brückenbildner für die Herstellung der Verknüpfung von Markierungsenzym mit dem Liganden auch die in den DE-A-21 28 743 und DE-A-22 60 185 beschriebenen Methoden sowie generell die Hydroxysuccinimidderivate geeignet.

Als immunologisch aktive Liganden kommen im Rahmen der Erfindung, wie schon erwähnt Antikörper, Fragmente derselben wie Fab, Fab2, Fc-Fragmente, chemisch erhaltene Derivate der Antikörper bzw. ihrer Fragmente, Antigene und Haptene wie Digoxin, Digoxigenin, T3, T4, Östradiol, Östriol, Progesteron, Folat, Theophyllin, Cortisol, Phenobarbital. und dergleichen in Betracht.

Die folgende Tabelle I zeigt die Ergebnisse einer T3-Enzymimmunobestimmung unter Verwendung eines herkömmlichen T3-POD-Konjugats (Konjugat I), eines erfindungsgemäß erhaltenen T3-POD-Konjugats (Konjugat II) und von radioaktiv markiertem T3 (RIA) als Referenzmethode. Verglichen werden ein Kontrollserum mit 7 Normalseren (NS) und 4 Störseren (STS; Problemseren). Verwendet wurden Humanseren.

## Tabelle  I

| Probe | Konjugat I (ng $T_3$/ml) | Konjugat II (ng $T_3$/ml) | RIA (Referenz) |
|---|---|---|---|
| Kontrollserum | 1,42 | 1,36 | n. d. |
| NS 1 | 0,95 | 0,95 | n. d. |
| NS 2 | > 6 | > 6 | >6,00 |
| NS 3 | 3,58 | 3,93 | 4,12 |
| NS 4 | 2,96 | 3,10 | 3,10 |
| NS 5 | 2,08 | 2,32 | 2,39 |
| NS 6 | 2,31 | 1,96 | 2,02 |
| NS 7 | 1,44 | 0,91 | 0,75 |
| STS 1 | 7,34 | 0,92 | 1,25 |
| STS 2 | 9,83 | 0,99 | 1,40 |
| STS 3 | 3,76 | 1,39 | 1,84 |
| STS 4 | > 6 | 1,17 | 1,20 |

n. d. = nicht durchgeführt

In der folgenden Tabelle II sind in analoger Weise die Ergebnisse der Digoxinbestimmung unter Verwendung eines Digoxin-POD-Konjugats, welches im einen Fall mit nativer POD, im anderen Fall mit erfindungsgemäß behandelter POD erzielt wurden. Zum Vergleich wurde ein RIA-Test mit radioaktiv markiertem Digoxin durchgeführt. Konjugat III ist erfindungsgemäß, Konjugat IV enthält nicht behandelte POD. Die Ergebnisse geben gefundene ng Digoxin pro ml an.

# Tabelle    II

| Kontrollserum | Dig. RIA | Konjugat III | Konjugat IV |
|---|---|---|---|
| Serum 1 | <0,5 | 0,38 | 1,76 |
| Serum 2 | <0,5 | 0,43 | 0,94 |
| Serum 3 | <0,5 | 0,43 | 0,81 |
| Serum 4 | 0,5 | 0,36 | 1,65 |
| Serum 5 | 0,85 | 0,86 | 3,53 |
| Serum 6 | <0,5 | 0,41 | 1,39 |
| Serum 7 | <0,5 | 0,40 | 1,20 |

Die obigen Werte zeigen, daß mit erfindungsgemäß hergestelltem POD-Konjugat Resultate erhalten werden, die den nach der RIA-Methode erhaltenen Ergebnissen entsprechen, während Konjugate mit nicht erfindungsgemäß behandelter POD zum Teil erhebliche Abweichungen vom richtigen Wert ergeben. Analoge Ergebnisse werden mit anderen Markierungsenzymen, die einen Kohlenhydratanteil im Molekül enthalten, erreicht.

Durch die Erfindung wird die Präzision und Zuverlässigkeit von Enzymimmunobestimmungen unter Verwendung von kohlenhydrathaltigem Markierungsenzym deutlich verbessert und die Schaffung von Testreagenzien ermöglicht, welche mit einer Enzymmarkierung ebenso zuverlässige Resultate erzielen wie mit radioaktiver Markierung und damit den Umgang mit radioaktiven Substanzen und die damit verbundenen Sicherheitsvorkehrungen unnötig machen.

Die folgenden Beispiele erläutern die Erfindung weiter.

Beispiel 1

Herstellung von Digoxigenin-POD

A) Oxidation und Reduktion von POD

10 mg handelsübliche POD in 1,5 ml bidest. Wasser pH 5,0 bei 0°C werden mit 0,2 ml 0,2 mol/l Natriumperjodat (42 mg/ml in bidest. Wasser) versetzt.

Nach 40 min. wird über Molekularsiebsäule (Sephadex® G-25) in 10 mmol/l Acetat pH 5,0 entsalzen.

Die proteinhaltigen Fraktionen werden vereinigt und auf 0°C abgekühlt.

Mit 1 mol/l Carbonat/Bicarbonat pH 9,0 wird auf pH 8,0 gestellt und sofort unter Rühren NaBH$_4$ ad 20 mmol/l zugegeben (≙ 0,756 mg/ml).

Nach 30 min. wird pH 8,5 gestellt. Dann wird weitere 2 Stunden bei 0°C gerührt. Anschließend erfolgt Dialyse gegen 0,1 mol/l KPO$_4$ pH 8,0. Das Produkt (POD(ox)) wird bei Bedarf an Sephacryl® S-200 in 0,1 mol/l Phosphatpuffer, pH 8,0 chromatographiert.

B) 200 mg des nach A) erhaltenen Produktes (POD(ox)) in 20 ml 0,2 mol/l Kaliumphosphat, pH 8,0 werden auf 4°C abgekühlt.

73 mg Digoxigenin-succinyl-OSu (OSu = Hydroxysuccinimid) werden in 5 ml abs. Ethanol gelöst und bei 4°C in einer Portion zur POD-Lösung gegeben. Dann wird 16 Stunden bei 4°C leicht gerührt.

Der Ansatz wird danach (ohne nennenswerten Aktivitätsverlust) gegen 0,1 mol/l K-Phosphat pH 8,0/0,15 mol/l NaCl dialysiert.

Die Aufreinigung des Ansatzes wird an einer 50 ml Phenylsepharose®-Säule vorgenommen. Äquilibrierung der Säule mit 0,1 mol/l K-Phosphat pH 8,0/ 0,15 mol/l NaCl. Vom Beginn des Aufziehens des Retentats mit 150 ml/h wird der Ablauf der Säule fraktioniert. Nach dem Aufziehen des Rohkonjugats wird mit dem Äquilibrierpuffer mit demselben Fluß weitereluiert. Ab ca. 50 bis 55 ml Volumen im Durchlauf wird das Säuleneluat vereinigt und der Konjugatpool mit einem Volumen von 50 bis 55 ml gebildet. Der Konjugatpool beinhaltet ca. 50 % der eingesetzten POD-Aktivität. Der Pool wird durch Ultrafiltration ad ca. 10 mg Konjugat/ml entsprechend ca. 10 KU/ml konzentriert.

Beispiel 2

Herstellung von $T_3$-POD

100 mg POD(ox), hergestellt nach Beispiel 1 A) in 0,1 mol/l Kaliumphosphatpuffer pH 8,0 bei 0°C mit 9,4 ml Dimethylformamid (DMF) versetzen. 25 mg BOC*-T3-OSu in 0,6 ml DMF bei 0°C zugeben. 18 Stunden unter Rühren weiterreagieren lassen. Alle Schritte lichtgeschützt.
*BOC = tert.-Butoxycarbonyl

Dialyse gegen 40 mmol/l Tris/HCl 7,5/0,15 mol/l NaCl bei 4°C. Retentat an 80 ml Phenylsepharose® (∅ 3 cm) in 40 mmol/l Tris/HCl pH 7,5/0,15 mol/l NaCl äquilibriert mit 1 SV**/h auftragen. Unumgesetzte POD läuft durch. Nach Erreichen der Ausgangsextinktion 40 mmol/l Tris/HCl pH 7,5/0,15 NaCl-Puffer ersetzen durch 50 % Ethylenglycol 40mmol/l.
**SV = Säulenvolumen

Tris pH 7,5/1 mol/l NaCl und Konjugat mit 1 SV/h eluieren.

Konjugatpeak poolen und Lösung bei 4°C mit 50 % des Konjugatvolumens 0,5 mol/l Hydroxylamin-Hydrochlorid-Lösung pH 7,0 versetzen und 2 Stunden rühren.

Dann Dialyse gegen 40 mmol/l $KPO_4$ pH 6,5 lichtgeschützt bei 4°C. Konjugat ad c = 10 mg/ml konzentrieren und mit RSA ad c = 5 mg/ml aufstocken.

Beispiel 3

Herstellung von $T_4$-POD

10 mg POD(ox), hergestellt nach Beispiel 1 A) in 1 ml 0,1 mol/l $KPO_4$ pH 8,5 langsam mit 0,94 ml DMF bei 0°C versetzen.

2,5 mg BOC-T4-OSu in 60 µl DMF unter Eiskühlung zugeben. Ansatz 6 Stunden bei 25°C rühren. Danach Dialyse gegen 40 mmol/l Tris/HCl pH 7,5/0,15 mol/l NaCl.

Aufreinigung des Dialysats an Phenylsepharose® in 40 mmol/l Tris/HCl pH 7,5/0,15 mol/l NaCl.

Pro 10 mg POD 4 ml Säulenvolumen verwenden. Elution mit Äquilibrierpuffer mit 1 SV/h. Wenn Ausgangsextinktion des Eluats wieder erreicht ist, wird das Konjugat mit 50 % Ethylenglycol in 40 mmol/l Tris/HCl pH 7,5/0,15 mol/l NaCl mit 1 SV/h eluiert.

Konjugatpeak wird ad c = 10 mg/ml konzentriert und gegen 40 mmol/l $KPO_4$ pH 6,5 bei 4°C lichtgeschützt dialysiert.

Konjugat wird ad c = 5 mg/ml mit RSA aufgestockt und bei 4°C gelagert.

Beispiel 4

Wie im Beispiel 1 B) beschrieben, jedoch unter Verwendung von nativer POD wird ein Digoxigenin-POD-Konjugat hergestellt. Das Konjugat wird gegen bidest. Wasser dialysiert und wie im Beispiel 1 A) beschrieben, der Oxidations-/Reduktionsbehandlung unterzogen, wobei lediglich anstelle der nativen POD eine äquimolare Menge des Digoxigenin-POD-Konjugats eingesetzt wird.

Das erhaltene Konjugat entspricht in seinen Eigenschaften im Test völlig dem nach Beispiel 1 erhaltenen Konjugat.

Beispiel 5

$T_3$-Test

1. Inkubation: Immunreaktion

Die mit anti-T3-Antikörpern beschichteten Kunststoffröhrchen der kommerziellen Testpackung "Enzymun Test® $T_3$" (Hersteller Boehringer Mannheim, Best.Nr. 204 528) werden mit 100 µl Standardlösung (Konzentrationsbereich 0 bis 6 ng T3/ml) bzw. Probe und je 1 ml T3-POD-Konjugatlösung versetzt.

Zur Anwendung kommen die Konjugate I-II (siehe Tabelle I) mit jeweils etwa 6 mU POD/ml in 0,12 mol/l Barbitalpuffer, pH 8,6, mit 0,04 % ANS (8-Anilinonaphthalinsulfonsäure). Die Reaktionszeit beträgt 2 Stunden bei Raumtemperatur. Danach saugt man das Inkubationsgemisch ab, mischt einmal mit Wasser und saugt nach spätestens 5 Minuten wieder ab.

2. Inkubation: Enzymatische Indikatorreaktion

Im Zeittakt der nachfolgenden photometrischen Messung (z. B. alle 15 Sekunden) pipettiert man in jedes Röhrchen je 1 ml der POD-Substratlösung (Phosphat/Citratpuffer 0,1 mol/l, pH 5,0, ABTS® 9,1 mmol/l. $H_2O_2$ 1,6 mmol/l) und läßt eine Stunde bei Raumtemperatur inkubieren. Anschließend wird die Extinktion bei Hg 405 nm gemessen und die Probenkonzentration an einer in Serie mitgeführten Standardkurve abgelesen.

**Patentansprüche**

1. Verfahren zur Herstellung eines für die Enzymimmunobestimmung geeigneten Konjugats aus einem Markierungsenzym, welches einen Kohlenhydratanteil enthält, wie Peroxidase und einer immunologisch wirksamen Substanz unter Anwendung einer am Proteinanteil des Markierungsenzyms angreifenden Kupplungsmethode, **dadurch gekennzeichnet,** daß man das Markierungsenzym vor oder nach der Kupplung mit der immunologisch wirksamen Substanz mit Perjodsäure oder einem Alkalisalz derselben in wäßrigem Medium oxidiert und das Oxidationsprodukt dann mit $NaBH_4$ reduziert.

2. Verfahren nach Anspruch 1, **dadurch gekennzeichnet,** daß man die Oxidation bei einem pH-Wert zwischen 4 und 8,5 in gepufferter Lösung unter Kühlen durchführt.

3. Verfahren nach Anspruch 1 oder 2, **dadurch gekennzeichnet,** daß man die Reduktion mit $NaBH_4$ bei einem pH- Wert zwischen 7,5 und 9 unter Kühlen durchführt.

4. Konjugat aus einem Markierungsenzym, welches einen Kohlenhydratanteil enthält und einer immunologisch wirksamen Substanz, **dadurch gekennzeichnet,** daß es durch Oxidation des Markierungsenzyms mit Perjodsäure oder einem Alkalisalz derselben in wäßrigem Medium und nachfolgende Reduktion mit $NaBH_4$ und Kupplung mit der immunologisch wirksamen Substanz unter Anwendung einer am Proteinanteil des Markierungsenzyms angreifenden Kupplungsmethode vor oder nach der Oxidations-Reduktionsbehandlung erhältlich ist.

5. Reagenz für die Enzymimmunobestimmung, **gekennzeichnet durch** einen Gehalt an Konjugat nach Anspruch 4.

**Claims**

1. Process for the preparation of a conjugate, suitable for the enzyme immune determination, of a labelling enzyme, which contains a carbohydrate part, such as peroxidase, and of an immunologically effective substance with the use of a coupling method acting on the protein part of the labelling enzyme, characterised in that, before or after the coupling with the immunologically effective substance, one oxidises the labelling enzyme with periodic acid or an alkali metal salt thereof in aqueous medium and then reduces the oxidation product with $NaBH_4$.

2. Process according to claim 1, characterised in that one carries out the oxidation at a pH value between 4 and 8.5 in a buffered solution, with cooling.

3. Process according to claim 1 or 2, characterised in that one carries out the reduction with $NaBH_4$ at a pH value between 7.5 and 9, with cooling.

4. Conjugate of a labelling enzyme, which contains a carbohydrate part, and of an immunologically effective substance, characterised in that it is obtainable by oxidation of the labelling enzyme with periodic acid or an alkali metal salt thereof in aqueous medium and subsequent reduction of the oxidation product with $NaBH_4$ and coupling with the immunologically effective substance, before or after the oxidation-reduction treatment, with the use of coupling method taking place on the protein part of the labelling enzyme.

5. Reagent for the enzyme immuno determination, characterised by a content of conjugate according to claim 4.

**Revendications**

1. Procédé de préparation d'un conjugué convenant à la détermination immunoenzymatique, conjugué constitué d'une enzyme de marquage, laquelle contient une partie d'hydrate de carbone, telle que la peroxydase et d'une substance à activité immunologique en utilisant un procédé de couplage agissant sur la partie protéique de l'enzyme de marquage, caractérisé en ce qu'on oxyde l'enzyme de marquage avant ou après le couplage avec la substance à activité immunologique, au moyen d'acide périodique ou d'un sel alcalin de celui-ci en milieu aqueux et en ce qu'on réduit ensuite le produit d'oxydation par $NaBH_4$.

2. Procédé selon la revendication 1, caractérisé en ce qu'on effectue l'oxydation à une valeur de pH entre 4 et 8,5 en solution tamponnée et en refroidissant.

3. Procédé selon la revendication 1 ou 2, caractérisé en ce qu'on effectue la réduction au moyen de $NaBH_4$ à une valeur de pH entre 7,5 et 9, en refroidissant.

4. Conjugué constitué d'une enzyme de marquage, laquelle contient une partie hydrate de carbone, et d'une substance à activité immunologique, caractérisé en ce qu'il peut être obtenu par oxydation de l'enzyme de marquage par l'acide périodique ou par un sel alcalin de celui-ci en milieu aqueux et par réduction subséquente du produit d'oxydation au moyen de $NaBH_4$ et couplage avec la substance à activité immunologique en utilisant un procédé de couplage agissant sur la partie protéique de l'enzyme de marquage avant ou après le traitement par oxydo-réduction.

5. Réactif pour la détermination immunoenzymatique, caractérisé par une teneur en conjugué selon la revendication 4.